# EUROPÄISCHE PATENTSCHRIFT

(11) **EP 0 787 713 B1**
(45) Veröffentlichungstag und Bekanntmachung des Hinweises auf die Patenterteilung: **13.06.2001**
(21) Anmeldenummer: 97100784.4
(22) Anmeldetag: 20.01.1997
(51) Int. Cl.: C07C 67/31, C07C 67/11, C07C 69/92, C07C 69/94

(54) **Verfahren zur Herstellung von aromatischen Methoxycarbonsäuremethylestern**
Process for the preparation of methyl esters of aromatic methoxycarboxylic acids
Procédé de fabrication d'esters méthyliques d'acides méthoxycarboxyliques aromatiques

(30) Priorität: 31.01.1996 DE 19603329
(43) Veröffentlichungstag der Anmeldung: 06.08.1997
(73) Patentinhaber: BAYER AG, 51368 Leverkusen (DE)
(72) Erfinder: Streicher, Willi, Dr., 47800 Krefeld (DE); Laakmann, Hans-Joachim, 42799 Leichlingen (DE)

(56) Entgegenhaltungen:
- EP-A- 0 640 582

## Beschreibung

Die vorliegende Erfindung betrifft ein besonders vorteilhaftes Verfahren zur Herstellung von aromatischen Methoxycarbonsäuremethylestern durch Methylierung von aromatischen Hydroxycarbonsäuren mit Dimethylsulfat.

Aromatische Methoxycarbonsäuremethylester sind wertvolle Synthesebausteine, z.B. zur Herstellung von Wirkstoffen und Polyestern. So ist z.B. 6-Methoxy-1-naphthoesäuremethylester wichtig als Zwischenprodukt zur Herstellung von Mitteln zur Vorbeugung und Behandlung von Spätfolgen des Diabetes mellitus (siehe EP-OS'en 59 596, 200 840 und 307 519 sowie US-PS 4 808 748) und 3-Methoxy-4-methylbenzoesäuremethylester als Zwischenprodukt zur Herstellung von Mitteln zur Behandlung von Asthma und Bronchitis (siehe EP-OS 83 228).

Grundsätzlich ist es bekannt, daß man aromatische Hydroxycarbonsäuren mit Dialkylsulfaten alkylieren kann (siehe z.B. Organikum, 15. Auflage, S. 252-255 und Houben-Weyl, 4. Auflage, Band 6/3, S. 62-66). Nachteilig dabei ist, daß man zur Erzielung nahezu vollständiger Umsätze und hoher Anteile von sowohl an der Hydroxyfunktion, als auch an der Carboxyfunktion alkylierten Produkten große Überschüsse an Dialkylsulfaten einsetzen muß (siehe z.B. Monatshefte für Chemie 91, 1077 (1960) - 6,4-fache molare Menge Dimethylsulfat und 75 % Ausbeute -, EP-PS 208 840 - 3,4-fache molare Menge Dimethylsulfat und 77 % Ausbeute an einem nur zu 87 % reinen Produkt - und Ber. Dt. Chem. Ges. 37, 3658 (1904)-2-fache molare Menge und 70 % Ausbeute).

Man kann auch geringere Mengen Dialkylsulfate einsetzen, muß dann aber andere Nachteile in Kauf nehmen. So eignet sich die in Ber. Dt. Chem. Ges. 40, 714 (1910) beschriebene Arbeitsweise, bei der man zunächst die Gesamtmenge der Reaktanden und Hilfsstoffe zusammengibt und dann auf Reaktionstemperatur erhitzt, nicht für eine Durchführung im technischen Maßstab, da die dabei plötzlich freiwerdende Reaktionswärme zu einem nicht kontrollierbaren und nicht reproduzierbaren Reaktionsablauf führt.

Das Verfahren der EP-OS 640 582 geht nicht von aromatischen Hydroxycarbonsäuren als solchen, sondern von Gemischen von aromatischen Hydroxycarbonsäuren mit aromatischen Methoxycarbonsäuren aus. Bei der praktischen Durchführung dieses Verfahrens geht man so vor, daß man zunächst aromatische Hydroxycarbonsäure mit Dimethylsulfat umsetzt, aus dem Reaktionsgemisch zuerst das Wunschprodukt (= aromatischer Methoxycarbonsäuremethylester), dann ein nur teilweise methyliertes Produkt (= aromatische Methoxycarbonsäure) abtrennt und die abgetrennte aromatische Methoxycarbonsäure dem nächsten Ansatz hinzufügt. Neben dem Aufwand, der für die Abtrennung und Rückführung der jeweiligen aromatischen Methoxycarbonsäure erforderlich ist, ist bei diesem Verfahren die Raum-Zeit-Ausbeute ungünstig. Bezogen auf neu eingesetzte aromatische Hydroxycarbonsäure beträgt die Ausbeute an aromatischem Hydroxycarbonsäuremethylester laut Beispiel 1 der EP-OS 640 582 zwar 96 %, bezogen auf die beiden Ausgangsmaterialien, aus denen aromatische Methoxycarbonsäuremethylester entstehen können (aromatische Hydroxycarbonsäure und aromatische Methoxycarbonsäure), sind es jedoch nur 63,7 %. Das Reaktionsgemisch enthält dann 36 % monoalkylierte aromatische Hydroxycarbonsäure (= aromatische Methoxycarbonsäure). Ähnlich sind die Verhältnisse bei den anderen Beispielen der EP-OS 640 582.

Es besteht also immer noch ein Bedürfnis nach einem Verfahren zur Herstellung von aromatischen Methoxycarbonsäuremethylestern, bei dem man gleichzeitig wenig Dimethylsulfat einsetzen, einfach arbeiten und das Zielprodukt in hohen Ausbeuten und Reinheiten erhalten kann.

Es wurde nun ein Verfahren zur Herstellung von aromatischen Methoxycarbonsäuremethylestern durch Methylierung der entsprechenden aromatischen Hydroxycarbonsäuren mit Dimethylsulfat in Gegenwart von Wasser und einer Base gefunden, bei dem man Wasser, Base und aromatische Hydroxycarbonsäure vorlegt, die 1- bis 2,5-fache molare Menge Dimethylsulfat (bezogen auf methylierbare Hydroxy- und Carboxygruppen) zudosiert und dabei den pH-Wert des Reaktionsgemisches durch Zugabe von wäßriger Base kontrolliert, das dadurch gekennzeichnet ist, daß man die Base in fein verteilter Form zugibt.

In das erfindungsgemäße Verfahren kann man z.B. aromatische Hydroxycarbonsäuren der Formel (I) einsetzen

HO-Ar-COOH (I),

in der
- Ar: für einen gegebenenfalls substituierten Phenyl- oder Naphthylrest steht
und so aromatische Methoxycarbonsäuremethylester der Formel (II) herstellen

CH₃O-Ar-COOCH₃ (II),

in der
- Ar: die bei Formel (I) angegebene Bedeutung hat.

Wenn es sich in den Formeln (I) und (II) bei Ar um Phenylreste handelt können diese gegebenenfalls z.B. 1 bis 2 gleiche oder verschiedene Substituenten enthalten. Wenn es sich in den Formeln (I) und (II) bei Ar um Naphthylreste handelt können diese gegebenenfalls z.B. 1, 2 oder 3 gleiche oder verschiedene Substituenten enthalten. Als Substituenten für Ar kommen z.B. Halogenatome, insbesondere Fluor, Chlor und Brom, Alkylgruppen, insbesondere solche mit 1 bis 4 C-Atomen, Alkylengruppen, insbesondere solche mit 2 bis 5 C-Atomen, Alkoxygruppen, insbesondere solche mit 1 bis 4 C-Atomen, Benzyloxygruppen, Halogenalkylgruppen, insbesondere solche mit 1 bis 3 Fluor-, Chlor- und/oder Bromatomen und 1 bis 4 C-Atomen, Nitrogruppen, Cyanogruppen und/oder Alkoxycarbonylgruppen, insbesondere solche mit 1 bis 4 C-Atomen im Alkoxyteil, in Frage. Die Substituenten können sich in verschiedenen Positionen am Rest Ar befinden und bei mehrkernigen Resten Ar in verschiedenen Positionen an einem oder mehreren Kernen angeordnet sein.

Beispiele für Alkylgruppen sind Methyl, Ethyl, n-Propyl, i-Propyl, n-Butyl, s-Butyl und t-Butyl. Beispiele für Alkylengruppen sind Vinyl, Allyl, Methallyl, 2-Butenyl und 1,1-Dimethylallyl. Beispiele für Alkoxygruppen sind Methoxy, Ethoxy, n-Propoxy, i-Propoxy, n-Butoxy, s-Butoxy und t-Butoxy. Beispiele für Halogenalkylgruppen sind Chlormethyl, Dichlormethyl, Trichlormethyl, Fluormethyl, Difluormethyl, Trifluormethyl, Chlordifluormethyl, Dichlorfluormethyl, 2,2,2-Trichlorethyl, 2,2,2-Trifluorethyl, 1,1-Dichlorethyl, 1,1-Difluorethyl, Perfluorethyl und Perchlorethyl. Beispiele für Alkoxycarbonylgruppen sind Methoxycarbonyl, Ethoxycarbonyl, i-Propoxycarbonyl, n-Propoxycarbonyl, n-Butoxycarbonyl, s-Butoxycarbonyl und t-Butoxycarbonyl.

Bevorzugt werden in das erfindungsgemäße Verfahren aromatische Hydroxycarbonsäuren der Formel (III) 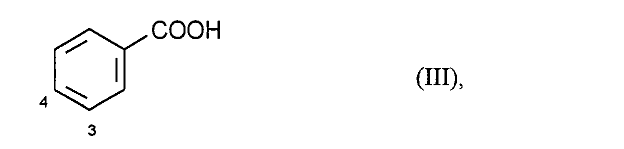 die in 3- oder 4-Position eine OH-Gruppe und an sonst unbesetzten Positionen gegebenenfalls 1 bis 2 C₁-C₄-Alkylgruppen, insbesondere Methylgruppen, enthalten und der Formel (IV) 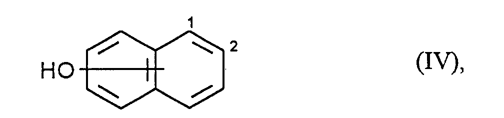 die in 1- oder 2-Position eine COOH-Gruppe und an sonst freien Positionen gegebenenfalls 1 bis 2 C₁-C₄-Alkylgruppen, insbesondere Methylgruppen, enthalten, eingesetzt und die entsprechenden aromatischen Methoxycarbonsäuremethylester hergestellt.

Ganz besonders bevorzugt wird 3-Hydroxy-4-methylbenzoesäsure oder 6-Hydroxy-1-naphthoesäure eingesetzt und 3-Methoxy-4-methylbenzoesäuremethylester oder 6-Methoxy-1-naphthoesäuremethylester hergestellt.

Die in das erfindungsgemäße Verfahren einzusetzenden aromatischen Hydroxycarbonsäuren sind bekannte Verbindungen oder können analog zu bekannten aromatischen Hydroxycarbonsäuren hergestellt werden.

Bezogen auf 1 mol eingesetzte aromatische Hydroxycarbonsäure kann man in das erfindungsgemäße Verfahren beispielsweise 75 bis 200 g Wasser einsetzen. Vorzugsweise beträgt diese Menge 100 bis 180 g. Das Wasser wird erfindungsgemäß auf zweierlei Weise zugefügt. Zum einen wird ein Teil des Wassers zusammen mit Base und aromatischer Hydroxycarbonsäure vorgelegt, zum andern wird wäßrige Base zur Kontrolle des pH-Werts des Reaktionsgemischs in fein verteilter Form dem Reaktionsgemisch zugegeben. Vorzugsweise arbeitet man so, daß man mindestens soviel Wasser vorlegt, daß das Reaktionsgemisch rührbar ist und man mindestens soviel Wasser der Base zur pH-Kontrolle zugibt, daß diese Base als wäßrige Lösung dem Reaktionsgemisch zugefügt werden kann.

Als Basen für das erfindungsgemäße Verfahren kommen beliebige, insbesondere wasserlösliche Basen in Frage, die in der Lage sind bei der jeweiligen aromatischen Hydroxycarbonsäure der Formel (I) die phenolischen und die Carbonsäure-Wasserstoffatome abzuspalten und die unter Reaktionsbedingungen keine störenden Nebenreaktionen verursachen. Insbesondere sind das solche Basen, die von Dimethylsulfat nicht methyliert werden. Bevorzugt sind anorganische Basen, insbesondere Alkalihydroxide wie Natrium- und Kaliumhydroxid. Die Basen gelangen vorzugsweise in Form einer wäßrigen Lösung zum Einsatz. Die Konzentration solcher wäßrigen Lösungen kann beliebig sein, beispielsweise zwischen 10 und 50 Gew.-% liegen.

Die Base wird insgesamt vorzugsweise mindestens in einer Menge zugesetzt, die ausreichend ist, um alle mit der eingesetzten aromatischen Hydroxycarbonsäure eingebrachten OH- und COOH-Gruppen zu deprotonieren. D.h. vorzugsweise setzt man pro mol aromatischer Hydroxycarbonsäure mindestens zwei Äquivalente Base ein. Nach oben hin kann die Basenmenge beispielsweise bis zu 3 Äquivalenten pro mol eingesetzte aromatische Hydroxycarbonsäure betragen. Bevorzugt ist der Einsatz von 2,01 bis 2,5 Äquivalenten Base pro mol eingesetzter aromatischer Hydroxycarbonsäure.

Beim erfindungsgemäßen Verfahren setzt man die Base auf zweierlei Weise zu. Einen Teil, z.B. mindestens 1 Äquivalent (bezogen auf aromatische Hydroxycarbonsäure), legt man vorzugsweise gemeinsam mit der aromatischen Hydroxycarbonsäure und Wasser vor. Der Rest der Base wird während-der Zudosierung des Dimethylsulfats als wäßrige Lösung in fein verteilter Form dem Reaktionsgemisch zugefügt und so der pH-Wert des Reaktionsgemisches kontrolliert.

Es ist vorteilhaft, den pH-Wert des Reaktionsgemisches so zu kontrollieren, daß die Zugabe von z.B. 60 bis 90 Gew.-%, vorzugsweise 70 bis 85 Gew.-% des insgesamt zum Einsatz kommenden Dimethylsulfats bei einem höheren und die Zugabe des restlichen Dimethylsulfats bei einem niedrigeren pH-Wert erfolgt. Welche pH-Werte bei welcher Hydroxycarbonsäure optimal sind kann erforderlichenfalls durch routinemäßige Reihenversuche ermittelt werden. Im allgemeinen erhält man gute Ergebnisse, wenn man den ersten Teil des Dimethylsulfats bei einem pH-Wert z.B. im Bereich 10 bis 13, vorzugsweise 10,5 bis 12,5 und den restlichen Teil des Dimethylsulfats z.B. bei einem pH-Wert z.B. im Bereich 7 bis 10, vorzugsweise 7,5 bis 9,5 zufügt. Weiterhin ist es vorteilhaft, den pH-Wert innerhalb der angegebenen Bereiche z.B. um nicht mehr als eine, vorzugsweise nicht mehr als 0,8 Einheiten variieren zu lassen.

Die Kontrolle des pH-Wertes während der Zugabe von Dimethylsulfat erfolgt erfindungsgemäß durch Zugabe von wäßriger Base in fein verteilter Form. Diese wäßrige Base kann z.B. eine 20 bis 50 gew.-%ige, vorzugsweise 40 bis 50 gew.-%ige wäßrige Lösung einer Base sein. Zur pH-Kontrolle während der Zugabe von Dimethylsulfat verwendet man zweckmäßigerweise die gleiche Basenzubereitung, die man auch für die gemeinsame Vorlage von Wasser, Base und aromatischer Hydroxycarbonsäure verwendet.

Dimethylsulfat wird vorzugsweise in einer Menge von 1,2 bis 2,1 mol pro mol aromatischer Hydroxycarbonsäure eingesetzt.

Eine feine Verteilung der wäßrigen Base bei der Kontrolle des pH-Wertes ist beispielsweise gegeben, wenn die wäßrige Base in Form von Tröpfchen zugesetzt wird, deren Durchmesser weniger als 1000 um beträgt. Dieser Durchmesser beträgt vorzugsweise weniger als 800 µm, insbesondere weniger als 500 µm. Man kann solche Tröpfchen erhalten, wenn man die wäßrige Base z.B. durch eine Düse zudosiert. Hierzu können die unterschiedlichsten Düsen verwendet werden. Als Beispiele seien Vollkegel-, Flachstrahl- und Zweistoffdüsen genannt.

Die Reaktionstemperatur beim erfindungsgemäßen Verfahren kann z.B. im Bereich 10 bis 90°C liegen. Vorzugsweise liegt sie zwischen 15 und 80°C, insbesondere bei 20 bis 70°C. Häufig ist es von Vorteil, das erfindungsgemäße Verfahren bei einer relativ niedrigen Temperatur zu beginnen, z.B. bei 10 bis 40°C, vorzugsweise 15 bis 35°C, und bei einer höheren Temperatur zu Ende zu führen, z.B. bei 30 bis 90°C, vorzugsweise bei 40 bis 80°C.

Im allgemeinen führt man das erfindungsgemäße Verfahren bei Normaldruck durch. Man kann auch bei erhöhtem oder erniedrigtem Druck arbeiten.

Die bisher beschriebene Arbeitsweise für das erfindungsgemäße Verfahren entspricht einer diskontinuierlichen Ausführungsform. Man kann das erfindungsgemäße Verfahren aber auch kontinuierlich durchführen, z.B. in der Weise, daß man es in einem Durchflußreaktor durchführt, in den man Dimethylsulfat und wäßrige Base (letztere in fein verteilter Form) in einer oder mehreren Zonen unter Kontrolle des pH-Wertes eindosiert.

Es ist vorteilhaft, während der Durchführung des erfindungsgemäßen Verfahrens für eine gute Durchmischung des Reaktionsgemisches zu sorgen.

Das nach der Durchführung des erfindungsgemäßen Verfahrens vorliegende Reaktionsgemisch kann auf an sich bekannte Weise aufgearbeitet werden, z.B. durch Filtration, Zentrifugieren, Phasentrennung, Extraktion, Destillation (auch Vakuum- und/oder Wasserdampfdestillation) und/oder nach dem Prinzip der pH-Trennung. Häufig erhält man schon ausreichend reine aromatische Methoxycarbonsäuremethylester, wenn man nach Reaktionsende den Rührer abstellt und die beiden Phasen sich trennen läßt. Die obere (organische) Phase enthält dann den hergestellten aromatischen Methoxycarbonsäuremethylester häufig in Ausbeuten von über 90 % der Theorie, bezogen auf eingesetzte methylierbare Verbindungen, und in Reinheiten von über 98 %.

Das erfindungsgemäße Verfahren hat eine Reihe von Vorteilen. Bei ihm wird das eingesetzte Dimethylsulfat besser ausgenutzt als bei anderen Verfahren. Partiell methylierte Produkte, z.B. aromatische Methoxycarbonsäuren, entstehen, wenn überhaupt, nur in Spuren. Deren spezielle Abtrennung und Rückführung ist deshalb nicht erforderlich. Man erhält aromatische Methoxycarbonsäuremethylester in hohen Ausbeuten und Reinheiten. Das erfindungsgemäße Verfahren ist auf einfache Weise durchführbar und ermöglicht hohe Raum-Zeit-Ausbeuten. Insgesamt betrachtet ist es also wesentlich günstiger als die bekannten Verfahren zur Herstellung von aromatischen Methoxycarbonssäuremethylestern.

### Beispiele

### Beispiel 1

In einem 8 m³-Reaktor wurden bei Raumtemperatur 1490 1 Wasser vorgelegt. Anschließend wurden 1198 kg 3-Hydroxy-4-methylbenzoesäure eingetragen und anschließend mit 50 %iger wäßriger Natronlauge ein pH-Wert von 11,5 eingestellt. Anschließend wurden unter Kühlen bei 20°C 1780 kg Dimethylsulfat im Verlaufe von 4 Stunden zulaufen gelassen und der pH-Wert durch Zugabe von 50 gew.-%iger wäßriger Natronlauge über eine Zweistoffdüse in Form von Tröpfchen eines Durchmessers von 200 µm zwischen 11,2 und 11,8 gehalten. Daran anschließend wurde das Reaktionsgemisch auf 55°C erwärmt und bei dieser Temperatur weitere 500 kg Dimethylsulfat innerhalb von 4 Stunden zudosiert, wobei der pH-Wert wie zuvor kontrolliert wurde, diesmal jedoch in einem Bereich von 8,2 bis 9,0. Nach Beendigung der Reaktion wurde der Ansatz ruhig stehengelassen, wobei sich zwei Phasen ausbildeten. Die untere wäßrige Phase wurde abgetrennt. Die obere organische Phase bestand aus 1362 kg 3-Methoxy-4-methylbenzoesäuremethylester. Das entspricht einer Ausbeute von 95 % der Theorie bezogen auf eingesetzte methylierbare Verbindungen. Der so erhaltene 3-Methoxy-4-methylbenzoesäuremethylester wies eine Reinheit von über 98 % (GC) auf.

### Beispiel 2

In einem 8 m³-Reaktor wurden bei Raumtemperatur 3 100 l Wasser vorgelegt, anschließend 1 450,4 kg p-Hydroxybenzoesäure eingetragen, dann mit 50 gew.-%iger wäßriger Natronlauge ein pH-Wert von 11,5 eingestellt und schließlich unter Kühlen bei 18 bis 25°C 1 560 kg Dimethylsulfat im Laufe von 4 Stunden zulaufen gelassen. Dabei wurde der pH-Wert mit feinstverteilter, über eine Zwei-Stoff-Zerstäuberdüse zudosierte Natronlauge zwischen 11,2 und 11,8 gehalten. Anschließend wurde das Reaktionsgemisch auf 55°C erwärmt. Bei dieser Temperatur wurden weitere 1 350,6 kg Dimethylsulfat in 4 Stunden zudosiert und auf die zuvor beschriebene Weise der pH-Wert zwischen 8,2 und 9,0 gehalten. Nach dem Ende der Reaktion ließ man sich die beiden Phasen trennen. Die obere organische Phase bestand aus 1 743 kg p-Methoxybenzoesäuremethylester. Das entspricht einer Ausbeute von 96 % der Theorie, bezogen auf eingesetzte methylierbare Verbindungen. Der so erhaltene p-Methoxybenzoesäuremethylester wies eine Reinheit von über 96 % (HPLC) auf und enthielt weniger als 0,1 Gew.-% p-Hydroxybenzoesäure und weniger als 0,1 % Paranissäure (= 4-Methoxybenzoesäure).

### Beispiel 3

In einem 5 l Dreihalskolben wurden bei Raumtemperatur 1,5 l Wasser vorgelegt und anschließend 461 g 6-Hydroxy-2-naphtoesäure eingetragen. Sodann wurde mit 50 gew.-%iger wäßriger Natronlauge ein pH-Wert von 11,5 eingestellt und dann unter Kühlen bei 18 bis 25°C 710 g Dimethylsulfat im Verlaufe von 6 Stunden zulaufen gelassen, wobei der pH-Wert durch Zudosieren feinstverteilter Natronlauge über eine Zwei-Stoff-Zerstäuberdüse zwischen 11,2 und 11,8 gehalten wurde. Nach dem Ende der Reaktion wurde das feste Produkt abgesaugt. So wurden 899,2 g 6-Methoxy-2-naphthoesäuremethylester erhalten. Das entspricht einer Ausbeute von 97,2 %, bezogen auf eingesetzte methylierbare Verbindungen. Der so erhaltene 6-Methoxy-2-naphthoesäuremethylester wies eine Reinheit von über 57 % (HPLC) auf und enthielt weniger als 0,1 Gew.-% 6-Hydroxy-2-naphthoesäure.

## Patentansprüche

1. Verfahren zur Herstellung von aromatischen Methoxycarbonsäuremethylestern durch Methylierung der entsprechenden aromatischen Hydroxycarbonsäuren mit Dimethylsulfat in Gegenwart von Wasser und einer Base, bei dem man Wasser, Base und aromatische Hydroxycarbonsäure vorlegt, die 1- bis 2,5-fache molare Menge Dimethylsulfat (bezogen auf methylierbare Hydroxy- und Carboxygruppen) zudosiert und dabei den pH-Wert des Reaktionsgemisches durch Zugabe von wäßriger Base kontrolliert, dadurch gekennzeichnet, daß man die Base in feinverteilter Form zugibt.

2. Verfahren nach Anspruch 1, dadurch gekennzeichnet, daß man aromatische Hydroxycarbonsäuren der Formel
HO-Ar-COOH (I),
in der
Ar für einen gegebenenfalls substituierten Phenyl- oder Naphthylrest steht
einsetzt und aromatische Methoxycarbonsäuremethylester der Formel (II)
CH₃O-Ar-COOCH₃ (II),
in der
Ar die bei Formel (I) angegebene Bedeutung hat,
herstellt.

3. Verfahren nach Anspruch 2, dadurch gekennzeichnet, daß es sich bei Ar um Phenylreste handelt, die gegebenenfalls 1 bis 2 gleiche oder verschiedene Substituenten enthalten oder um Naphthylreste, die gegebenenfalls 1, 2 oder 3 gleiche oder verschiedene Substituenten enthalten, wobei die Substituenten ausgewählt sind aus der Gruppe der Halogenatome, der Alkylgruppen mit 1 bis 4 C-Atomen, der Alkylengruppen mit 2 bis 5 C-Atomen, der Alkoxygruppen mit 1 bis 4 C-Atomen, der Benzyloxygruppen, der Halogenalkylgruppen mit 1 bis 3 Fluor-, Chlor- und/oder Bromatomen und 1 bis 4 C-Atomen, der Nitrogruppe, der Cyanogruppe und der Alkoxycarbonylgruppen mit 1 bis 4 C-Atomen im Alkoxyteil.

4. Verfahren nach Ansprüchen 1 bis 3, dadurch gekennzeichnet, daß man bezogen auf 1 mol eingesetzte aromatische Hydroxycarbonsäure 75 bis 200 g Wasser einsetzt.

5. Verfahren nach Ansprüchen 1 bis 4, dadurch gekennzeichnet, daß man mindestens soviel Wasser vorlegt, daß das Reaktionsgemisch rührbar ist und mindestens soviel Wasser der Base zur pH-Kontrolle zugibt, daß diese Base als wäßrige Lösung dem Reaktionsgemisch zugefügt werden kann.

6. Verfahren nach Ansprüchen 1 bis 5, dadurch gekennzeichnet, daß man insgesamt 2 bis 3 Äquivalente Base pro mol aromatische Hydroxycarbonsäure einsetzt.

7. Verfahren nach Ansprüchen 1 bis 6, dadurch gekennzeichnet, daß man mindestens 1 Äquivalent der Base gemeinsam mit aromatischer Hydroxycarbonsäure und Wasser vorlegt.

8. Verfahren nach Ansprüchen 1 bis 7, dadurch gekennzeichnet, daß man 60 bis 90 Gew.-% des zum Einsatz kommenden Dimethylsulfats bei einem pH-Wert im Bereich 10 bis 13 und das restliche Dimethylsulfat bei einem pH-Wert von 7 bis 10 zufügt.

9. Verfahren nach Ansprüchen 1 bis 8, dadurch gekennzeichnet, daß man die wäßrige Base in Form von Tröpfchen zusetzt, deren Durchmesser weniger als 1000 µm beträgt.

10. Verfahren nach Ansprüchen 1 bis 9, dadurch gekennzeichnet, daß man es bei Temperaturen im Bereich 10 bis 90°C durchführt.

## Claims

1. Process for the preparation of an aromatic methoxycarboxylic acid methyl ester by methylation of the corresponding aromatic hydroxycarboxylic acid with dimethyl sulphate in the presence of water and a base, in which the water, base and aromatic hydroxycarboxylic acid are initially introduced into the reaction vessel, 1 to 2.5 times the molar amount of dimethyl sulphate (based on the methylatable hydroxyl and carboxyl groups) are metered in and, during this procedure, the pH of the reaction mixture is controlled by addition of an aqueous base, characterized in that the base is added in finely divided form.

2. Process according to Claim 1, characterized in that an aromatic hydroxycarboxylic acid of the formula
HO-Ar-COOH (I),
in which
Ar represents an optionally substituted phenyl or naphthyl radical,
is employed and an aromatic methoxycarboxylic acid methyl ester of the formula (II)
CH₃O-Ar-COOCH₃ (II),
in which
Ar has the meaning given in the case of formula (I),
is prepared.

3. Process according to Claim 2, characterized in that Ar are phenyl radicals which optionally contain 1 or 2 identical or different substituents, or naphthyl radicals which optionally contain 1, 2 or 3 identical or different substituents, the substituents being chosen from the group consisting of halogen atoms, alkyl groups having 1 to 4 C atoms, alkylene groups having 2 to 5 C atoms, alkoxy groups having 1 to 4 C atoms, the benzyloxy group, halogenoalkyl groups having 1 to 3 fluorine, chlorine and/or bromine atoms and 1 to 4 C atoms, the nitro group, the cyano group and alkoxycarbonyl groups having 1 to 4 C atoms in the alkoxy part.

4. Process according to Claims 1 to 3, characterized in that 75 to 200 g of water are employed per mol of aromatic hydroxycarboxylic acid employed.

5. Process according to Claims 1 to 4, characterized in that at least an amount of water such that the reaction mixture is stirrable is initially introduced, and at least an amount of water is added to the base for controlling the pH such that this base can be added to the reaction mixture as an aqueous solution.

6. Process according to Claims 1 to 5, characterized in that a total of 2 to 3 equivalents of base are employed per mol of aromatic hydroxycarboxylic acid.

7. Process according to Claims 1 to 6, characterized in that at least one equivalent of the base is initially introduced together with the aromatic hydroxycarboxylic acid and water.

8. Process according to Claims 1 to 7, characterized in that 60 to 90% by weight of the dimethyl sulphate employed is added at a pH in the range from 10 to 13 and the remaining dimethyl sulphate is added at a pH of 7 to 10.

9. Process according to Claims 1 to 8, characterized in that the aqueous base is added in the form of droplets, the diameter of which is less than 1000 µm.

10. Process according to Claims 1 to 9, characterized in that it is carried out at temperatures in the range from 10 to 90°C.

## Revendications

1. Procédé de préparation d'esters méthyliques d'acides méthoxycarboxyliques aromatiques par méthylation des acides hydroxycarboxyliques aromatiques correspondants avec du sulfate de diméthyle en présence d'eau et d'une base, dans lequel on introduit initialement l'eau, la base et l'acide hydroxycarboxylique aromatique, on dose de 1 à 2,5 fois la quantité molaire de sulfate de diméthyle (rapporté aux groupes hydroxyle et carboxyle pouvant être méthylés), et on contrôle ainsi le pH du mélange réactionnel par addition d'une base aqueuse, caractérisé en ce qu'on ajoute la base sous forme finement divisée.

2. Procédé selon la revendication 1, caractérisé en ce qu'on introduit des acides hydroxycarboxyliques aromatiques de formule (I)
HO-Ar-COOH (I),
dans laquelle
Ar représente un radical phényle ou naphtyle éventuellement substitué, et on prépare des esters méthyliques d'acides méthoxycarboxyliques de formule (II)
CH₃O-Ar-COOCH₃ (II)
dans laquelle
Ar a la signification donnée dans la formule (I).

3. Procédé selon la revendication 2, caractérisé en ce que Ar est un radical phényle éventuellement substitué par 1 ou 2 substituants identiques ou différents, un radical naphtyle éventuellement substitué par 1, 2 ou 3 substituants identiques ou différents, les substituants étant choisis dans le groupe constitué par des atomes d'halogène, des groupes alkyle ayant 1 à 4 atomes de carbone, des groupes alkylène ayant 2 à 5 atomes de carbone, des groupes alcoxy ayant 1 à 4 atomes de carbone, le groupe benzyloxy, des groupes halogénoalkyle ayant 1 à 3 atomes de fluor, de chlore et/ou de brome et 1 à 4 atomes de carbone, des groupes nitro, des groupes cyano et des groupes alcoxycarbonyle ayant 1 à 4 atomes de carbone dans la partie alcoxy.

4. Procédé selon les revendications 1 à 3, caractérisé en ce qu'on emploie 75 à 200 g d'eau par mole d'acide hydroxycarboxylique aromatique employé.

5. Procédé selon les revendications 1 à 4, caractérisé en ce qu'on introduit initialement au moins une quantité d'eau telle que le mélange réactionnel peut être agité est introduit initialement, et qu'on ajoute au moins une quantité d'eau à la base pour contrôler le pH de manière que cette base puisse être ajoutée au mélange réactionnel sous forme d'une solution aqueuse.

6. Procédé selon les revendications 1 à 5, caractérisé en ce qu'on emploie 2 à 3 équivalents de base par mole d'acide hydroxycarboxylique aromatique.

7. Procédé selon les revendications 1 à 6, caractérisé en ce qu'on introduit initialement au moins un équivalent de la base avec l'acide hydroxycarboxylique et l'eau.

8. Procédé selon les revendications 1 à 7, caractérisé en ce qu'on ajoute 60 à 90% en poids de sulfate de diméthyle employé à un pH dans la plage de 10 à 13 et qu'on ajoute le sulfate de diméthyle restant à un pH de 7 à 10.

9. Procédé selon les revendications 1à 8, caractérisé en ce qu'on ajoute la base aqueuse sous forme de gouttelettes dont le diamètre est inférieur à 1000 µm.

10. Procédé selon les revendications 1 à 9 qui est effectué à des températures dans la plage de 10°C à 90°C.
